# EUROPEAN PATENT SPECIFICATION

(11) **EP 3 183 576 B1**
(45) Date of publication and mention of the grant of the patent: **13.11.2019**
(21) Application number: 15754181.4
(22) Date of filing: 20.08.2015
(51) Int. Cl.: G01N 33/543, C12M 1/32, C12M 1/00

(54) **METHOD FOR COATING A SOLID SUPPORT**
VERFAHREN ZUR BESCHICHTUNG EINES FESTEN TRÄGERS
PROCÉDÉ DE REVÊTEMENT D'UN SUPPORT SOLIDE

(30) Priority: 20.08.2014 EP 14181625
(43) Date of publication of application: 28.06.2017
(73) Proprietor: Eppendorf AG, 22339 Hamburg (DE)
(72) Inventor: PLUESTER, Wilhelm, 22359 Hamburg (DE); REMACLE, Jose, 5100 Jambes (BE); ALEXANDRE, Isabelle, 5340 Haltinne (BE); PLENNEVAUX, Christelle, 5640 Saint Gerard (BE); DE LONGUEVILLE, Francoise, 5101 Erpent (BE)
(74) Representative: Uexküll & Stolberg
(86) International application number: PCT/EP2015/069150
(87) International publication number: WO 2016/026932

(56) References cited:
- US-A- 5 278 063
- US-A1- 2012 171 769
- FLORIS ET AL: "Dynamic rearrangement of disulfide bridges influences solubility of whey protein coatings", INTERNATIONAL DAIRY JOURNAL, ELSEVIER APPLIED SCIENCE, BARKING, GB, vol. 18, no. 5, 8 December 2007 (2007-12-08), pages 566-573, XP022526384, ISSN: 0958-6946
- ALAN TIN-LUN LAM ET AL: "Cationic Surface Charge Combined with Either Vitronectin or Laminin Dictates the Evolution of Human Embryonic Stem Cells/Microcarrier Aggregates and Cell Growth in Agitated Cultures", STEM CELLS AND DEVELOPMENT, vol. 23, no. 14, 15 July 2014 (2014-07-15) , pages 1688-1703, XP055167671, ISSN: 1547-3287, DOI: 10.1089/scd.2013.0645

## Description

The present invention relates to a method for coating a solid support which is suitable for being used in cell culture applications. The method comprises a step in which a solution of a carrier protein, which comprises attached thereto or as part of its amino acid sequence at least one cell function-modulating peptide sequence, is heated to at least 50°C and incubated under conditions which result in the formation of soluble carrier protein aggregates comprising between 2 and 1000 protein molecules. Subsequently, the protein aggregates are brought into contact with the solid support under conditions that allow for the non-covalent adsorption of the aggregates to the support. The carrier protein is an albumin or a protein derived from an albumin. The invention also relates to a coated solid support having non-covalently adsorbed on its surface at least one of the above-mentioned carrier protein aggregates.

### BACKGROUND OF THE INVENTION

In vitro cell cultures are commonly performed in plastic cell culture vessels, such as polystyrene flasks or dishes. Such flasks or dishes are highly stable at routinely used culture temperatures of 37°C or higher, and they are moreover transparent and easy to manufacture. However, most of the cells or cell lines which are currently available for cell culturing do not show sufficient attachment to the hydrophobic plastic surface. Accordingly, the results achieved by growing cells on unmodified plastic surfaces are often poor.

To overcome the problem with insufficient cell attachment to plastic cell culture flasks or dishes, several methods were proposed in the prior art to render the surface of these items more hydrophilic. For example, a plasma treatment was carried out which provides reactive hydrophilic groups on the surfaces. The surface-modified products obtainable by these techniques, however, have been proven unsuitable for certain applications, e.g. the culturing of stem cells or highly differentiated cells. In addition, the modification of a surface by plasma treatment is expensive and laborious. Accordingly, alternative methods were developed for providing surface-modified culture vessels.

It has early been recognized in the field of cell culturing that the use of components of the extracellular matrix (ECM) can greatly influence the ability of a surface to bind viable cells. In vivo, cells are in interaction with each other and with their environment mainly through the binding of structural proteins which form part of the ECM. Adhesive interactions of cells with the ECM were found to influence a number of important cellular activities, including cell growth, cell differentiation, cell migration, and tissue organization. In fact, the ECM is involved in a number of complex physiological processes like morphogenesis, angiogenesis, wound healing, and tumor metastasis.

The ECM is a three-dimensional molecular complex that varies in composition, and includes bioactive proteins, such as laminin and fibronectin, glycoproteins, hyaluronic acid, elastins, proteoglycans, and collagens. Cells interact with the ECM trough binding of specific surface receptors which are expressed on their surface. The most prominent cell surface receptors which are known to interact with components of the ECM are the integrins. In vertebrates, 24 integrin subtypes were found, each one exhibiting a different ligand specificity. These integrin subtypes are summarized in Barczyk et al. 2010 (Cell Tissue Res 339, 269-280) and in Yurchenco & Patton 2009 (Curr Pharma Des. 15, 1277-1294). The binding of some integrins to their ECM target components is effected by specific binding sequences in the ECM molecule. One prominent binding sequence which has been identified in many matrix proteins, e.g. in fibronectin, laminin and osteopontin, is the peptide sequence Arg-Gly-Asp (RGD) which has been described by Pierschbacher and Ruoslahti, J. Biol Chem 1987, 262, 17294-17298. To enhance the attachment of cultured cells to support items like flasks or microtiter plates, it has been contemplated in the prior art to graft material derived from the ECM to the surface of such items.

In a first approach, it was proposed to provide the surface of culture vessels with complete ECM material. Submucosa derived from small intestine, stomach or urinary bladder tissue was used as a source of the ECM. See, for example, US 5,281,422, US 5,554,389, US 6,099,567, and US 6,206,931. Matrigel™, a gelatinous protein mixture which is widely used in the field of cell culturing and which is available for cell culture applications from manufacturers like Corning Life Sciences, is another suitable source for complete ECM material. Matrigel™ is secreted by the murine Engelbreth-Holm-Swarm (EHS) sarcoma and contains a plethora of growth factors and other biologically active molecules which form a natural, biocompatible environment for cultured cells. However, items coated with complete ECM materials like Matrigel™ lack defined characteristics as it remains unknown which of the different ECM components are part of the mixture and are accessible after coating. Also, the culturing of cells on surfaces that contained whole ECM extracts turned out to be problematic owing to the fact that it is not possible to tightly control the cell signaling cascades that are initiated by binding of the cells to the distinct ECM components.

In view of these problems, it was contemplated to provide more tailored surfaces which have been coated with individual ECM proteins or ECM protein fragments. However, the production of full-length ECM proteins is expensive and technically cumbersome. To overcome these problems, it was suggested to graft smaller peptides which harbor cell-binding sequence motifs, such as the RGD motif, onto support surfaces to improve cell attachment. Peptides can easily be synthesized in high numbers, and they are relatively stable under conditions of grafting. However, both the coating of full-length proteins and peptides suffer from an insufficient stability of the coated products. Conventional techniques for protein or peptide immobilization on the surface of a support item rely on the non-covalent adsorption of the protein or peptide to the surface. Unfortunately, however, desorption of the protein or peptide under cell culture conditions, in particular during incubation at 37°C, have been found to result in the detachment of the cultured cells from their support which may ultimately lead to the induction of programmed cell death (apoptosis) in these cells.

One way of preventing the problem of protein desorption is to use covalent chemical fixation of the proteins or peptides to hydroxyl, amine or other reactive groups on the surface of the support material. Direct covalent coupling can be achieved by surface activation using chemicals such as tresyl chloride, glutaraldehyde, or cyanoborohydrid which provides for the generation of functional groups, such as amine, hydroxyl, carboxyl, or aldehyde groups. Subsequently, these groups are reacted with functional groups in the proteins or peptides which results in a covalent coupling. Such coupling methods are extensively described, e.g. in US 5,278,063, US 2005/0059140, and US 7,399,630. However, cultured cells are very sensitive to the presence of chemical compounds. Even very low amounts of reactive chemicals can significantly inhibit or even abolish cell growth or attachment. Therefore, the chemical fixation of proteins or peptides to cell culture surfaces is undesirable.

It can be seen from the above that there is a need for new methods that provide for a stable immobilization of proteins or peptides on surfaces of cell culture items such as vessels, flasks, plates and dishes. The demands for protein or peptide coatings applied to cell culture surfaces are quite high. The coatings should be stable and no or very low amounts of immobilized protein or peptide should be released when incubated at 37°C for periods of between 1 to 10 days. In addition, for use in cell culture applications, the surfaces normally need to be sterile. The present invention provides a novel method which fulfills these needs and provides additional advantages as well.

### DESCRIPTION OF THE INVENTION

The present invention provides a method for coating a solid support item with carrier proteins. The method is rapid, highly reproducible, and easy to implement into existing production processes of cell culture consumables. By incorporating cell function-modulating peptide sequences into the carrier proteins, it is possible to tailor a synthetic surface which is capable of modulating the certain functions of the cells which are cultured on such surface, e.g. by promoting adhesion, growth, migration and/or differentiation. The method disclosed herein provides products which are highly suited for use in cell culture applications, as it allows to produce coatings which are stable and uniform. In addition, the method allows the productions of coatings which are characterized by a diversity of cell function-modulating peptide sequences which can be specifically tailored for certain applications. Finally, the method of the invention does not use any reactive chemicals that could interfere with downstream applications of the coated surfaces.

In a first aspect, the invention relates to a method for coating a solid support which is suitable for being used in cell culture applications. The method of the invention comprises the following steps:
(a) preparing a solution of at least one carrier protein comprising, either attached thereto or as a part of its amino acid sequence, at least one cell function-modulating peptide sequence wherein said cell function-modulating peptide sequence comprises a cell-binding peptide motif, and wherein said carrier protein is an albumin or a protein derived from an albumin;
(b) heating the solution to at least 50°C and incubating same under conditions which result in the formation of soluble carrier protein aggregates comprising between 2 and 1000 protein molecules;
(c) contacting the carrier protein aggregates with the solid support under conditions that allow for the non-covalent adsorption of the aggregates to the support;
(d) optionally, washing the solid support to remove unbound carrier protein and carrier protein aggregates;
whereby a solid support is provided that comprises on its surface a plurality of cell function-modulating peptide sequences. The method of the invention preferably does not include the use of reactive chemicals for coupling the carrier proteins to the support items.

The method of the invention is hence directed to providing a coating of protein or peptide-containing material onto solid support items which are suitable for use in cell culture applications. The support items can be of any material, wherein the coating of glass or plastic surfaces is particularly preferred herein. The solid support which is coated by applying the method of the invention can be the surface of a product which is commonly used in cell culture applications, in particular a cell culture vessel, such as a cell culture flask, a roller bottle, a culture plate, a tube, a microtiter plate, a membrane or a culture dish. In a preferred embodiment of the invention, the solid support is a cell culture plate with 6, 12, 24 or 48 wells. These plates are available from a number of different manufacturers, e.g. the plates which are marketed by Fisher Scientific under the trade name Corning™. In another preferred embodiment, the solid support can be a small plastic disk or beads which are routinely used as substrates in a biofermentor for culturing adherently growing cell lines. Such discs made of polypropylene are available, for example, from Eppendorf AG under the trade name Fibra-Cel®. In a further embodiment, the solid support can be a larger carrier plate used as a substrate in a biofermentor for culturing adherently growing cell lines. Another solid object that can be subjected to the coating method of the invention is a medical implant which shall be seeded with cells before implantation into the patient. The surface of the solid support to be coated with the protein material can be either hydrophilic or hydrophobic which means that a large variety of items can be subjected to the method of the invention to create surfaces that become seeded by cells. The method avoids any kind of aggressive chemical treatment of the surface which could affect subsequent cell growth.

The invention is to some extent based on the insight that cell function-modulating peptide sequences, such as peptide sequences which provide for cell attachment, can be adsorbed to solid supports, e.g. to cell culture vessels, by coupling them to a carrier protein or, alternatively, including them by recombinant methods into the amino acid sequence of said carrier protein. The carrier proteins are then heated to induce partial denaturation and the formation of soluble protein aggregates which can be adsorbed to the surface of the support. Upon heating, the carrier proteins within the aggregates become partially denatured but remain soluble which allows the uniform distribution of the protein aggregates on the surface to be coated. In contrast, completely denatured proteins would result in the formation of precipitated protein patches, thereby leading to an undesirable local accumulation of proteins rather than a uniform distribution. According to the invention, the coating of the carrier proteins on the support item in turn leads to a uniform distribution of the cells which are subsequently cultured on the surface of the support. A uniform distribution of cells is an important objective to be achieved in a number of cell culture applications. Methods for evaluating the uniform distribution of cells on a surface have been described, e.g. in Amenta et al. (1998), Proceedings of the 25th Annual Conference on Computer Graphics and Interactive Techniques, 415-421.

The carrier proteins which are used for the method of the invention can be of any size, but it is preferred that the carrier proteins have a molecular weight of at least 20,000 or more. In an even more preferred embodiment of the invention, the carrier protein has a size of at least 30,000, 40,000, 50,000, 60,000, 70,000, 80,000, 90,000, 100,000 or more. A size of the carrier protein of at least 50,000 is particularly preferred for use in the method of the invention. According to the invention, the carrier protein to be employed in the method of the invention is an albumin, such as human serum albumin bovine serum albumin, or egg albumin.

The carrier proteins used in the method of the invention have been modified to include one or more cell-function modulating peptide sequences. According to the invention, the carrier proteins are used for providing a suitable environment for the cell function-modulating peptide. The cell function-modulating peptide sequence can be associated to the carrier protein by means of a chemical coupling or by recombinant techniques. According to a preferred embodiment, the carrier protein is a recombinant protein which comprises, as part of its amino acid sequence, one or more cell function-modulating peptides. According to an even more preferred embodiment, the carrier protein is a recombinant albumin protein, such as bovine or human serum albumin into which one or more cell function-modulating peptides have been inserted.

According to another embodiment, the carrier protein comprises 1 or more, 2 or more, 3 or more, 4 or more, 5 or more, or even 10 or more cell function-modulating peptides. In yet another embodiment, the carrier protein comprises 1 cell function-modulating peptide.

The cell function-modulating peptides are coupled to the carrier proteins or integrated into their primary structure so that upon heat-induced aggregate formation, the cell function-modulating peptides, which are present on different carrier proteins, form a molecular mosaic of cell function-modulating sequences. Such array consisting of different or identical peptides provides for a strong interaction with numerous receptors or binding molecules on the surface of the cell. Thus, in a preferred embodiment of the invention, the cell function-modulating peptides are comprised by the carrier proteins so as to form a molecular mosaic of cell function-modulating peptides which are present on or in the different carrier proteins of the aggregate.

The presence of 2, 3, 4, 5, 6, or more identical or different cell function-modulating peptides per carrier protein is particularly preferred according to the invention.

A high number of cell function-modulating peptide sequences, either identical or different, within the same aggregate in the form of a molecular mosaic of peptides which are available for cell binding is of particular interest for cell culturing applications. The cells to be cultured possess numerous receptor molecules on their surface which interact with the surface to which they attach during proliferation. The molecular mosaic of cell function-modulating peptides which is provided by the aggregates on the surface of an item that was coated by the method of the invention ensures that these cell function-modulating peptides a presented in a sufficiently high density to modulate the function of cells that have attached to the aggregates.

The cell function-modulating peptide sequence can be incorporated into the carrier protein by replacing amino acids of the native carrier protein with amino acids of the cell function-modulating peptide which are located in the same or a similar position of the tertiary structure within the native protein from which the cell function-modulating peptide originates. The conformation of the carrier protein which includes one or more cell function-modulating peptides can be calculated and visualized by computer programs such as Swiss-PdbViewer, ICM-Browser, Phyre, Modeller, HHpred, Robetta or the BioInfoBank server.

As used in the context of the invention, the term "cell function-modulating peptide" refers to any peptide sequence which, upon binding to a cell, is able to modulate one or more cell functions, such as cell proliferation, cell differentiation, cell migration, cell activation, cell ageing, or cell apop-tosis. Also included by the term are peptides which provide for the attachment of the cell to a surface which is coated with such peptides. Numerous peptide sequences which are capable of modulating certain cell functions are known in the art. The selection of the cell function-modulating peptide or the combination of cell function-modulating peptides to be coated onto the support will determine the ability of the coated support item to promote adhesion, growth, movement, or differentiation of cultured cells on the substrate. Also included within the meaning of the term "peptides" are peptide mimetics, e.g. mimetics which contain one or more non-naturally occurring amino acids.

According to a preferred embodiment of the invention, the support is coated with a carrier protein that comprises one or more peptide sequences, the latter of which provide for cell anchorage, i.e. the attachment of cells to the coated surface through binding of the peptide sequences. Preferably, the coated support comprises at least one carrier protein comprising one or more of the peptide sequences depicted in SEQ ID NO:1-26. These sequences contain cell binding motifs which are derived from ECM components, such as laminin, fibronectin and collagen. According to a particularly preferred embodiment of the invention, the solid support is coated with at least one carrier protein comprising one or more peptide sequences which provide for cell anchorage and include an RGD sequence motif. The RGD sequence is preferably part of a loop structure of the carrier protein. In another embodiment, the peptide sequence which provides for cell anchorage is a cyclic peptide. According to a particularly preferred embodiment of the invention, the solid support is coated with different carrier proteins, each of which comprising a different peptide sequence which provides for cell anchorage and include the RGD sequence motif.

In another preferred embodiment, the support is coated with at least one carrier protein comprising one or more peptide sequences which modulate cell growth and differentiation. Peptide sequences which have been reported to have growth factor activity are set forth in SEQ ID NO:27-56. The sequences in SEQ ID NO:27-56 contain motifs which are known from different growth factor molecules, amongst others basic fibroblast growth factor (bFGF), transforming growth factor α (TGF-α), nerve growth factor (NGF), osteopontin, epidermal growth factor (EGF), vascular endothelial growth factor (VEGF) and platelet-derived growth factor (PDGF).

According to the invention, all peptides disclosed in NO:1-56 can be used either in the linear form or in cyclic form (i.e. the cyclis peptides may be used for embodiments that are based on the chemical coupling of the peptides to their carrier proteins. Therefore, where the sequence listing refers to a cyclic peptide, this reference is to be understood as referring also to the linear form of this peptide and vice versa.

In another preferred embodiment, the support is coated with at least one carrier protein comprising one or more peptide sequences which have been found to be effective for promoting growth and differentiation of osteogenic cells. These peptides include the sequences shown in SEQ ID NO:1, 13, 15, 22 and 49. In another preferred embodiment, the support is coated with at least one carrier protein comprising one or more peptide sequences which have been found to be effective for promoting growth and differentiation of neuronal cells. These peptides include the sequences shown in SEQ ID NO:1, 5-7, 17, 24-25, 27-29 and 55-56. In another preferred embodiment, the support is coated with at least one carrier protein comprising one or more peptide sequences which have been found to be effective for promoting growth and differentiation of hepatocytes. These peptides include the sequences shown in SEQ ID NO:1, 6, 16-19, 27-32, and 34.

According to the invention, the support item can be coated with only a single species of a cell function-modulating peptide or with a mixture of different modulating peptides. In a preferred embodiment, however, 2, 3, 4, 5 or more different cell function-modulating peptide sequences are used, either in the same or in different carrier protein. Hence, it is preferred that the surface of the support is coated with carrier protein aggregates comprising at least 2 different cell function-modulating peptides, wherein at least 3, at least 4, at least 5, at least 6, at least 7, at least 8, at least 9, or at least 10 different peptides are likewise possible. The method of the invention can also provide for a coating that includes 10, 20 or 30 different cell function-modulating peptides. For example, it is possible to use a first cell function-modulating peptide which provides for cell anchorage in combination with a second peptide which promotes cell differentiation. The first and the second cell function-modulating peptides can be included into the primary structure of the same or different carrier protein molecules. For example, if BSA is used as a carrier protein, it will be possible to produce a recombinant BSA molecule which includes in its primary structure (a) a peptide of any of SEQ ID NO:1-26 and (b) a peptide of any of SEQ ID NO:27-56.

Alternatively, it might be desirable for certain applications to produce different recombinant BSA molecules, each of which comprises only a single cell function-modulating peptide in its primary structure. The different BSA carrier proteins which are characterized by the possession of a specific cell function-modulating peptide may then be mixed when preparing the carrier protein solution in step (a) of the method described herein.

The combination of the cell function-modulating peptides which are attached or included into the sequence of the carrier protein can be tailored to achieve or suppress certain cell functions. In one embodiment, the specific composition of the different cell function-modulating peptide sequences coated on the surface of the solid support facilitates cell growth. In another embodiment, the composition of the cell function-modulating peptide sequences induces or facilitates cell activation, including the activation of one or more kinase enzymes. Kinase activation can involve, e.g. a tyrosine kinase, a Src tyrosine kinase and activation of the Ras-MAPK pathway. In still another embodiment, the composition of the different peptide sequences contained is inhibitory for cell division and/or cell differentiation. In still another embodiment, the surface of the support item has been coated with a peptide or a combination of peptides which prevents delamination of the cells from the coated surface.

In an alternative embodiment, the cell function-modulating peptides can be chemically coupled to the respective carrier protein. Methods for coupling small peptides to larger proteins are known in the art. For example, as described in more detail in the below example, the cell function-modulating peptides can be coupled to a carrier protein, i.e. an albumin, by using an amine-to-sulfhydryl crosslinker for creating sulfhydryl-reactive, maleimide-activated carrier proteins that may be used for coupling to cell function-modulating peptides. Other methods for coupling peptides to proteins can be used as well. Such methods are described, e.g. in G.T. Hermanson "Bioconjugate techniques", 3rd ed. (2013) Academic Press; ISBN-13: 978-0123822390.

In a first step of the coating method of the invention, a solution of the carrier protein is prepared which means that the proteins are dissolved in an aqueous medium such as water or an aqueous buffer, e.g. phosphate buffer. The amount of carrier protein will be adapted to avoid any precipitation of the protein from the solution. Where different carrier proteins are used for the coating, these carrier proteins are preferably mixed into a single solution before heating. Alternatively, separate solutions may be prepared for each of the carrier proteins, and the subsequent heating step is carried out for each of the carrier protein solutions separately. Depending on the size and the nature of the carrier protein, the amount of the carrier protein to be included into the solution may vary. Generally, the amount of the carrier protein to be used in the solution of step (a) of the method of the invention will be in the range of 1-50 mg/ml, for example 5-40 mg/ml, 10-30 mg/ml, or 20-25 mg/ml.

Optionally, the solution containing the carrier proteins is sterilized after step (a). As most cell culturing applications require culturing under sterile conditions, contaminations in the protein solution used in step (a) must be avoided. Techniques for providing sterile protein solution are commonly known in the art and include, e.g. filtration of the protein solution through a filter membrane having a pore size in the range of 0.1 to 0.5 µm, preferably between 0.2 and 0.3 µm, more preferably 0.22 µm. Suitable filter membranes can be purchased from a number of different manufacturers, e.g. from Sartorius, Millipore, Pall and others. The method of the invention avoids the use of sterilizing chemicals or physical processes like UV or gamma irradiation which affect the integrity of peptides and proteins.

In a subsequent step of the method of the invention, the solution containing the carrier protein is heated to a temperature that induces the formation of soluble protein aggregates which comprise between 2 and 1000 protein molecules per aggregate on average. Preferably, the aggregates resulting from method step (b) comprise between 2 and 1000 protein molecules per aggregate, and most preferably between 5 and 100 protein molecules per aggregate. As used herein, the formation of aggregates comprising between 2 and 1000 protein molecules means that at least 50%, and preferably at least 80%, at least 90%, even more preferably at least 95%, of the carrier proteins in the solution are present in an aggregated comprising between 2 and 1000 protein molecules. The temperature used during the heating step will be at least 50°C, wherein higher temperatures are more preferred, e.g. at least 55°C, at least 60°C, at least 65°C, at least 70°C. Stated differently, the protein solution is heated in step (b) to a temperature between 50°C and 75°C, preferably, between 65°C and 73°C, and more preferably between 68°C and 72°C. The heating step will be performed for a time period of between 10 minutes and 48 hours, wherein in most cases the high temperature will be applied for 1-24 hours, e.g. 2h, 4h, 6h, 8h, 10h, 12h, 14h, 16h, 18h, 20 or 22h. The optimum temperature to be used in step (b) of the method will vary with the size and the nature of the carrier protein, and also with the composition of the solution, e.g. the concentration of salts or buffers which are added. The skilled person will readily be able to determine for a given carrier protein solution the temperature and the incubation time which are required to form soluble aggregates containing the recited number of protein molecules.

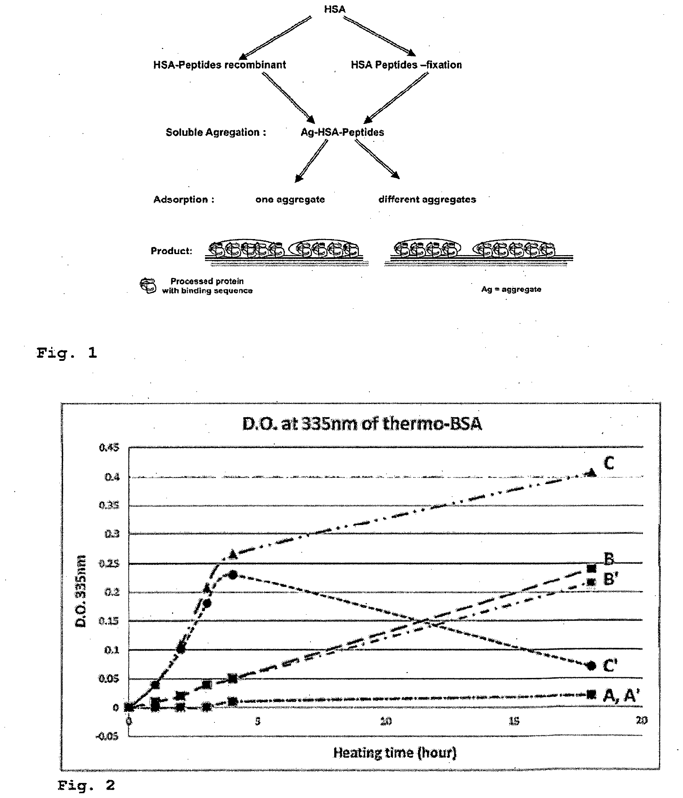

A rather simple approach for monitoring the solubility of the aggregates is to measure the optical density (OD) of the protein solution at a wavelength of 335 nm. The OD will increase with the formation of protein aggregates in the solution. As shown in figure 2, it is easy to determine the most appropriate temperature for aggregate formation. As shown in this figure, BSA was heated to different temperatures (65°C, 70°C, and 75°C) for different periods of time and the formation of BSA aggregates was monitored by measuring the OD at 335 nm. While a temperature of 65°C was evidently too low to induce the formation of BSA aggregates (see A, A' in figure 2), a temperature of 75°C was too high, as the latter induced precipitation of a major part of the aggregates from the solution, as evidenced by a drop of the OD value after removing precipitated proteins by centrifugation (see C' in figure 2). For BSA, a temperature of 70°C was found to be particularly advantageous, as only a small fraction of the aggregates that have formed upon heating for about 18h precipitated from the solution upon centrifugation (see B' in figure 2). Thus, it is possible to determine conditions which result in the formation of soluble carrier protein aggregates by using routine methods known to the skilled person. Preferably, the formation of aggregates is monitored by measuring the OD at 355 nm. A high OD at 335 nm which remains high after centrifugation indicates a strong protein aggregation and a low level of precipitation (or no precipitation). Once the optimum conditions have been determined, they can be routinely used for the production of soluble aggregates of the respective carrier protein.

Likewise, it is also easily possible to determine the average number of protein molecules which are comprised by an aggregate. For this purpose, a fraction of the protein solution which has been heated to induce aggregate formation is subjected to native polyacrylamide gel electrophoresis (PAGE) under non-reducing conditions. Such electrophoresis will provide information regarding the molecular weight of the protein aggregates formed upon heating. Once the molecular weight of the carrier protein is known, the number of proteins in the aggregate can be calculated.

Preferably, the conditions in step (b) of the method of the invention are selected such that at least 50%, preferably at least 60%, at least 70%, at least 80%, at least 90%, or at least 95% of the protein aggregates in the solution are maintained in soluble form.

The heating step (b) can be performed in a standard thermomixer device. A stirring rate of between 100-500 rpm can be used during the incubation at increased temperature.

After heating of the solution is completed, the carrier protein aggregates are brought into contact with the solid support under conditions that allow for the non-covalent adsorption of the aggregates to the support. For this, the solution containing the protein aggregates can be centrifuged, e.g. for 5 minutes at 1,000 to 5,000 rpm to remove any insoluble and precipitated protein material. The supernatant obtained from the centrifugation step is then applied to the support item to be coated. In one embodiment, a fraction of the solution is pipetted directly to the surface of the support item, and the support item is subsequently dried at a temperature of 25-37°C until the liquid has completely evaporated from the surface of the support item. Drying can also be carried out in a standard evaporator. In an alternative embodiment, the protein aggregates obtained in step (b) are lyophilized and then dissolved in an aqueous medium which is then applied to the support. The incubation time in step (c) is in the range of 1 to 48 hours, preferably between 2 and 24 hours, such as 4 hours, 6 hours, 8 hours, 10 hours, 12 hours, 14 hours, 16 hours, 18 hours or 20 hours.

Optionally, the support item which has been subjected to coating will be washed after step (c) to remove any unbound carrier protein and carrier protein aggregates. Washing can be performed by rinsing the surface of the support item with water or a suitable buffer, such as phosphate buffer. The washing buffer should preferably not contain any denaturing compounds or detergents such as SDS to avoid removal of the adsorbed aggregates from the surface of the support item. After washing and drying the support item can be stored until its further use for cell culture.

When conducting the method as set out above, a solid support is provided comprising on its surface a plurality of cell function-modulating peptide sequences which are present in non-covalently adsorbed carrier protein aggregates. Preferably, the surface of the coated solid support comprises on average between 1000 and 1,000,000, preferably between 100,000 and 400,000 cell function-modulating peptide sequences per mm² surface area.

In a further aspect, the invention relates to a coated solid support which is obtainable by a method as described above.

In a still further aspect, the invention relates to a coated solid support having non-covalently adsorbed on its surface at least one aggregate of carrier proteins, wherein said carrier proteins are albumins or proteins derived from albumins and comprise, either attached thereto or as a part of their amino acid sequence, at least one cell function-modulating peptide sequence, and wherein said aggregate comprises between 2 and 1000 protein molecules and preferably between 5 and 100, more preferably between 50-100.

As stated elsewhere herein, the solid support can be of any material, wherein support items having a surface of glass or plastic are particularly preferred. The solid support can be the surface of a cell culture item, e.g. a cell culture vessel, such as a cell culture flask, a roller bottle, a culture plate, a culture tube, a microtiter plate a membrane, a disk, a bead, a slide, a carrier plate for a biofermentor, or a culture dish. In a preferred embodiment of the invention, the solid support of the invention is a cell culture plate with 6, 12, 24 or 48 wells.

The solid support of the invention comprises on its surface at least one aggregate of the carrier proteins described elsewhere herein. The aggregated carrier proteins provide for a molecular mosaic of cell-function modulating peptide sequences. Thus, in a preferred embodiment, the surface of said support comprises a molecular mosaic of cell-function modulating peptide sequences which are present on or in the different carrier proteins of the aggregate. The carrier protein is does not naturally occur in the extracellular matrix.

The surface of the coated support of the invention may comprise only one of the above discussed cell function-modulating peptide sequences, but it is preferred that it comprises two or more different cell function-modulating peptide sequences, either in one or in more than one carrier protein. For example, the surface of the support item may comprise at least 3, at least 4, at least 5, at least 6, at least 7, at least 8, at least 9, or at least 10 different cell function-modulating peptide sequences. In a particular embodiment, the surface of said support comprises a carrier protein aggregate comprising a molecular mosaic of cell-function modulating peptide sequences available for the cell binding. The mosaic formed can comprise identical or different cell-function modulating peptide sequences. This is of particular interest in view of the fact that a high number of receptors is present on the surface of the cells to be cultured and their possible interactions in promoting or suppressing cell function. In a particularly preferred aspect, the coated solid support comprises at least one cell function-modulating peptide sequence with a cell-binding peptide motif, preferably a RGD motif. For example, the support may comprise one or more of the cell-binding peptides selected from SEQ ID NO:1-26 and/or one or more peptides with growth factor activity selected from SEQ ID NO:27-56.

The carrier proteins which comprise the cell function-modulating peptide sequences attached thereto or included therein are fixed to the surface of the solid support by non-covalent adsorption. Although the cell function-modulating peptide sequences are non-covalently adsorbed to the surface of the support item of the invention, the coating provided by the method of the invention is highly stable. Preferably, less than 30% of the carrier proteins are removed from the coated surface after incubation for 24h at 37°C, more preferably less than 25%, less than 20%, less than 15%, less than 10%, or less than 5% in PBS or a cell culture medium. Even more preferably, less than 4%, 3%, 2%, 1% or less than 0.5% or 0.1% of the carrier proteins detach from the surface under these conditions. Detachment of the non-covalently adsorbed protein can of course be achieved by abrasive chemicals such as organic solvents or detergents.

According to the invention, it will also be possible to attach cell-function modulating peptides after aggregation of the carrier proteins. It was found that carrier proteins which have been provided with cell-function modulating peptides only after aggregation showed similar results as proteins to which these peptides had been attached before aggregation when tested with RGD peptides on HEK293 cells for 15 min in cell culture.

In a particularly preferred embodiment, the surface of the solid support comprises between 1000 and 1,000,000, preferably between 100,000 and 400,000 cell function-modulating peptide sequences per mm² surface area.

The surface of the support item of the present invention may comprise at least 5 or more, and preferably at least 10 or more locations or spots which have been coated by different carrier proteins, e.g. with BSA proteins that differ from each other by the respective cell function-modulating peptide sequence.

### DESCRIPTION OF THE FIGURES

Figure 1 shows the principle of the method of the invention. Illustrated are the different preparation methods for solid surface coatings and the resulting products.
Figure 2 shows the effect of incubation time and temperature on the aggregation of BSA. Albumin solutions were heated to different temperatures (65°C, 70°C, and 75°C) for different periods of time, and the formation of BSA aggregates was monitored by measuring the optical density (OD) at 335 nm. The solutions were then centrifuged, and the OD at 335 nm was measured again. 65°C before (A) and after centrifugation (A'), 70°C before (B) and after centrifugation (B'), 75°C before (C) and after centrifugation (C').

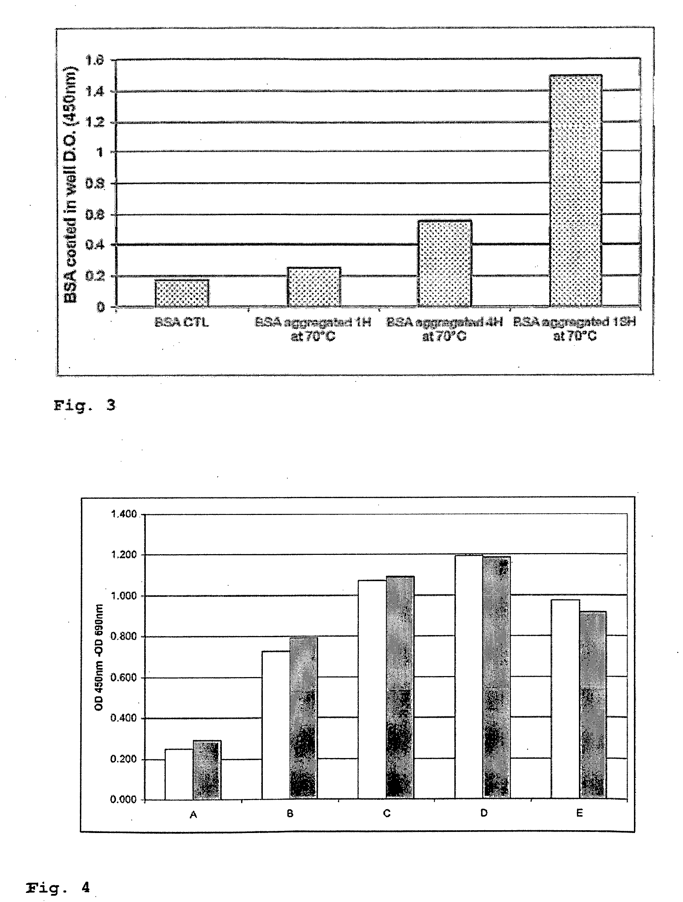

Figure 3 shows the efficiency of coating of aggregated BSA onto polystyrene plates. The graph shows the amount of BSA coated to polystyrene wells after incubation with a solution of BSA aggregated at 70°C for different periods of time (1h, 4h 18h). BSA CTL indicates heated BSA which was not subjected to heating.
Figure 4 shows a comparison of the coating efficiency on hydrophobic and hydrophilic surfaces. 24 plates were provided which had previously been treated for cell culture (tissue culture treated (TCT), Costar®, Corning #3524) or not (Costar®, Corning #3738). The wells of these plates were coated for 4h with 1 mg/ml aggregated BSA which had been coupled to linear or cyclic RGD as described in example 2. After washing, the plates were dried at 37°C. 150,000 HEK293 cells were inserted into each well and incubated for 15 min in culture medium with 10% FCS. After 15 min incubation, the attached cell number was estimated by colorimetric assay using the WST-1 reagent. The absorbance was measured at 450 nm using a reference at 690 nm. White bar = TCT; grey bar = non-treated surface. A: BSA alone, B: BSA+SMCC, C: BSA+SMCC+RGD linear, D: BSA+SMCC+RGD cyclic, E: no coating.

### EXAMPLES

### Example 1: Construction of BSA carrier proteins having inserted into their sequence cell function-modulating peptide sequence

Different recombinant human serum albumin (HAS) proteins were constructed which comprise as part of their primary structure a cell function-modulating peptide sequence that was derived from an ECM protein. For this purpose, the conformation of the cell function-modulating peptide sequence within the native ECM protein was determined using the software 3D Molecule Viewer, which is a component of the Vector NTI software (Invitrogen). In a subsequent step, the conformation of the native ECM protein was compared to that of the HAS protein to identify regions having a similar conformation. Where similar regions were identified, the amino acids of the BSA located in the selected region were replaced by the amino acids of the cell function-modulating peptide, and the resulting recombinant protein structure was recalculated using the program "SWISS-MODEL". In this way, three loops were identified in the native HAS protein which are suitable for being replaced by the cell function-modulating peptide sequence of SEQ ID NO:2 (GRGDS).

The first loop (loop-1) contains the amino acids 80 to 84 (DESAE) of native HAS (SEQ ID NO:57). The second loop (loop-2) contains the amino acids 116 to 120 (AKQEP) of native HAS, and the third (loop-3) amino acids 194 to 198 (QAADK). The amino acids of these loops were replaced by the sequence of SEQ ID NO:2 (GRGDS). The resulting constructs having the cell function-modulating peptide sequence of SEQ ID NO:2 included into their loop region 1, 2 or 3 of HAS are shown in SEQ ID NO:58-60, respectively. A construct having the cell function-modulating peptide sequence of SEQ ID NO:2 included into all three loop regions of HAS is depicted in SEQ ID NO:61.

In another construct, the sequence REDV was incorporated into a linear stretch of the BSA protein located between amino acids 137 and 140 (PRLV). The resulting construct is shown in SEQ ID NO:62. In yet another construct, the peptide RYVVLPR was incorporated into an inversed loop of the BSA protein which is located between amino acids 129 and 135 (HKDDNPN). The resulting construct is shown in SEQ ID NO:63. A construct comprising RGD incorporated into the loop3, REDV incorporated into the linear stretch, and RYVVLPR incorporated into the inversed loop is shown in SEQ ID NO:64. The different constructs having 1, 2 or 3 cell function-modulating peptide sequences in their sequence were used as further described below. A VSV-G tag or a His tag was added to the C terminal sequence for affinity purification. The expression was performed in *Pichia pastoris.* The expression was followed by SDS-Page electrophoresis with the identification of the band at 68 kD as the expressed HAS compared to the non transfected cells.

### Example 2: Construction of BSA carrier proteins by attachment of cell function-modulating peptide sequences to the BSA surface

A solution of 10 mg HAS (Sigma #021M7353V) in 1 ml of 100 mM phosphate buffer at pH 7.4 was incubated with 3 mg of Sulfo-SMCC (#MF158668, Thermo Scientific, Rockford, Il, USA) for 1.5h at 21°C. The solution was dialyzed overnight in the same buffer. Coupling of the peptide was performed by incubating 1 mg activated HAS with 1 mg peptide overnight at 21°C. RGD cyclic or spacer peptides were synthetically synthesized by Eurogentec (Liege, Belgium) or Bachem AG (Bubendorf, Switzerland) . The peptides were suspended in PBS solution with 10 mM EDTA. The same method was used for coupling the peptides on the aggregated protein.

### Example 3: Protein aggregation

A solution containing 10 mg BSA (Sigma #A3059) in 1 ml of 100 mM phosphate buffer at pH7.4 was incubated in a low bind tube (Eppendorf AG, Hamburg, Germany) at 70°C, for 4h on a ThermoMixer® (Eppendorf AG, Hamburg, Germany) with a mixing rate of 500 rpm. After incubation, the optical density (OD) of the solution was determined at 335 nm in order to determine the presence of protein aggregates.

### Example 4: Determination of the experimental conditions for the obtaining soluble protein aggregates

A solution containing 10 mg BSA (Sigma #A3059, Saint Louis, USA) and 1 µg biotinylated BSA (Sigma #A8549, Saint-Louis, USA) in 1 ml of 100 mM phosphate buffer at pH 7.4 was prepared. The solution was divided into aliquots in Eppendorf® Protein LoBindTubes (Eppendorf, Hamburg, Germany) and heated at different temperatures and for different periods of time on a ThermoMixer® (Eppendorf, Hamburg, Germany) with a mixing rate of 500 rpm. After incubation, the OD was determined at 335 nm to monitor the formation of protein aggregates. The OD of the native soluble BSA was 0 at this wavelength. Higher OD values indicate the formation of protein aggregates. The solutions were then centrifuged for 5 min at 5000 rpm and the OD was measured once again. Centrifugation removes insoluble aggregates from the solution.

The results are given in figure 2. The OD of the solution heated at 70°C for 4h was 0.04 and identical after centrifugation. The solution heated at 75°C for 18h showed an OD of 0.407 at 335 nm but only an OD of 0.07 after centrifugation. This shows that part of the proteins were denatured by heating and precipitated from the solution.

The heated protein solutions were separated on a non-denaturing electrophoresis blue native acrylamide gel (gradient from 4.5% to 16%). Electrophoresis conditions were as follows: 18h migration at 4 mA between cathode buffer: 50 mM Tricine, 15 mM Bis-Tris pH 7, 0.02% Coomassie Blue and anode buffer of 50 mM Bis-Tris pH 7. The solution of aggregated BSA incubated at 70°C for 4h showed a majority of aggregates with a molecular weight between 600,000 and 1 million with only a small amount of aggregates having a lower molecular weight. The protein solution incubated at 65°C for 4h showed low molecular weight BSA aggregates (mostly dimers to 8-mers). The solution incubated at 75°C did not show any band since the protein aggregates and/or precipitates were too large to enter the gel. The above shows how to determine the optimum parameters of temperature and time for achieving aggregate formation without substantial loss of protein material.

### Example 5: Coating of aggregated BSA in polystyrene wells

A solution containing 10 mg BSA (Sigma #A3059, Saint Louis, USA) and 1 µg biotinylated BSA (Sigma #A8549, Saint-Louis, USA) in 1 ml of 100 mM phosphate buffer at pH 7.4 was prepared. The solution was divided into aliquots in Eppendorf® Protein LoBindTubes (Eppendorf, Hamburg, Germany) and heated at 70°C for 0h, 1h, 4h and 18h on a ThermoMixer® (Eppendorf, Hamburg, Germany) with a mixing rate of 500 rpm. After the heating step, the different aggregated BSA solutions were put in contact with the polystyrene wells of a "tissue culture treated (TCT)" multiwell plate (Corning #3595, NY, USA). 50 µl/well (500 µg BSA + 50 ng of biotinylated-BSA/well) were used for coating.

The wells were sealed with plastic film and incubated at room temperature for 18h. After incubation, the wells were emptied, and washed for 1 x 5 min followed by 2 x 1 min with 200 µl of PBS, then incubated for 30 min at room temperature with 50 µl of streptavidin-peroxidase conjugate solution (1 µg/ml of STAV-HRP in 100 mM phosphate buffer + 0.5% of skim milk).

After incubation of conjugate, wells were washed 3 x 1 min at room temperature with 200 µl PBS and incubated for exactly 5 min at room temperature with 50 µl TMB solution. The reaction was stopped by addition of 50 µl of 1N HCl solution in each well, and the plate was read in a Multiskan™ plate reader (LabSystems, ThermoScientific) at a wavelength of 450 nm. Plots of the optical density of the different BSA solutions are depicted in figure 3. It can be seen that the amount of coated aggregates increases with the time used for the aggregation.

The coated plates were then incubated for 24h in PBS solution at 37°C to measure the amount of protein which is released from the plates. It was found that under these conditions, no biotinylated BSA could be detected in the PBS solution, which means that no protein is released from the coatings.

Direct streptavidin-peroxidase assays were conducted for detecting the presence of biotinylated BSA on the surface of coated wells before and after washing with aqueous PBS solution for 24 h at 37°C. The wells were coated by three different conditions of aggregation (1h, 4h, 18h at 70°C). Biotinylated BSA was measured by determining the optical density as explained above. The results are shown in the below Table 1. It can be seen that the amount of biotinylated BSA was essentially identical before and after washing for all of the three aggregation conditions. There was no loss of biotinylated BSA after 24 h washing at 37°C. These results show that the coating of aggregated BSA is stable and does not dissolve in aqueous media. Non-aggregated BSA was used as control (CTL).

**Table 1: Effect of washing of coated BSA aggregates**

| | Not washed | Washed (24h) |
|---|---|---|
| CTL | 0.17 | 0.16 |
| Aggregation 1h at 70° | 0.25 | 0.33 |
| Aggregation 4h at 70° | 0.56 | 0.60 |
| Aggregation 18h at 70° | 1.54 | 1.64 |

### Example 6: Culturing of cells on hydrophobic or hydrophilic surface

HEK293 Cells were cultivated in 24 plates that had been either treated for cell culture (TCT, Costar® Corning #3524) or not (Costar® Corning #3738). 150,000 cells were inserted into each wells and incubated for 15 min in culture medium with 10% FCS. Coating of the well was performed for 4h with aggregated BSA at 1 mg/ml as in example 3 and coated on the wells as in example 5. After coating, the plates were washed and dried at 37°C. A linear or cyclic RGD-containing peptide was coupled to BSA using SMCC (as described in Example 2). After 15 min of incubation, the number of attached cells was estimated by colorimetric assay using the WST-1 reagent (N°10008883, Cayman Chemical, Michigan, US), and the absorbance was read at 450 nm using a reference at 690 nm.

The results are shown in figure 4. It can be seen that in the control experiment, TCT increases the number of cells that attached to a surface compared to non-treated surface (E). The same efficiency of cell attachment was achieved between TCT and non-TCT plates when using plates that had been coated with aggregated BSA comprising a linear RGD-containing peptide (C) or BSA comprising a cyclic RGD-containing peptide (D). A coating that does not comprise any cell function-modulating peptide has a negative effect on cell attachment (A).

### Examples 7: Stem cell differentiation

Several peptides or peptide combination were coupled to BSA to provide carrier proteins. The protein solutions were heated to obtain aggregates as described in example 3. Subsequently, the protein aggregates were coated to the surface of a culture flask as explained in example 5. Human mesenchymal stem cells (hMSC) were obtained from Lonza (#PT-2501, Vervier, Belgium). The cells were first grown in a stem cell growth medium (BulletKit™, Lonza PT-3001) for 1-2 days before inducing differentiation.

Cell cultivation for osteogenic differentiation: The cells were seeded to the wells of the coated plates at day 0 at a density of 3.7 x 10³ cells/cm. The induction of differentiation was started at day 1 using the hMSC Osteogenic Differentiation BulletKit™ (Lonza, PT-3002). The induction medium was refreshed at day 3, 6 and 10. The assays were performed at day 14. Differentiation of osteoblasts results in matrix maturation and mineralization. Osteoblasts contain mineralized nodules composed of hydroxyapatite and organic components including type 1 collagen; the hydroxyapatite was assayed by the Osteolmage™ Bone Mineralization Assay (Lonza, PA-1503). The binding of Osteolmage™ to the hydroxyapatite results in a fluorescent signal which can be observed and measured. Other markers of differentiation are the synthesis of pro-collagen 1, TGF-β and fibronectin. The cell cultures were tested after 14 and 21 days of differentiation. A combination of the peptides of SEQ ID NO:1 and 13, or peptides SEQ ID NO:1 and 15 was particularly efficient both for cell growth and cell differentiation. The RGD-containing, aggregated BSA resulted in much better cell growth than TCT in the pre-differentiation step and at higher differentiation. The degree of differentiation was similar to that observed with the BioCoat™ fibronectin-coated plates (BD, Belgium).

Cell cultivation for adipogenic differentiation: The hMSC cells were seeded to the wells at day 0 at a density of 2.1 x 10⁴ cells/cm (day 0). The culture medium was hMSC Adipogenic Differentiation BulletKit™ (Lonza, PT-3004), and the culture was performed according to the instructions of the manufacturer. The culture medium was refreshed at day 4. The induction of the differentiation was started at day 5 using the hMSC Adipogenic Differentiation BulletKit™ (Lonza, PT3004). At day 8, the induction medium was replaced by maintenance medium for 3 days. Another cycle of induction was started at day 11 and a third one at day 15. Maintenance medium was added at day 18, 20, 22, 25 and the assays performed at day 26. The aggregated BSA which comprises the peptide of SEQ ID NO:1 allowed good cell growth and differentiation into adipogenic cells.

### Example 8: Neuronal differentiation of stem cells.

The cyclic RGD-containing peptide of SEQ ID NO:1 was coupled to BSA to provide a carrier protein and heated to obtain aggregates as described in example 3. The aggregates were then coated on a culture vessel surface as explained in example 5.

Stem cells were Human Neural Stem Cells (H-9 hNSC) obtained from Gibco® (# N7800-200) (Life Technologies, Belgium). They are derived from the NIH approval H9 (WA09) embryonic stem cells. The culture medium and the differentiation medium were from Gibco®. The cells were first grown for 2 days in a stem cell growth medium (StemPro® NSC SFM, Life Technolgies) before induction of differentiation according to the protocol recommended by the manufacturer.

Several culture surfaces were used for the differentiation step: standard tissue culture treated (TCT) surface, Synthemax® (Corning, Belgium), PureCoat™ ECM Mimetic Cultureware Fibronectin Protein (Becton Dickinson, BD, Belgium), BioCoat™ Poly-L-Ornithine/Laminin (BD, Belgium) and surfaces coated with the RGD-comprising BSA aggregates prepared according to the present invention.

### Example 9: Culture in serum-free medium

The cyclic RGD-containing peptide of SEQ ID NO:1 was coupled to HSA to provide a carrier protein and heated to obtain aggregates as described in examples 3. The aggregates were then coated on a culture vessel surface as explained in example 5.

Stem cells were Human Mesenchymal Stem Cells (hMSC) obtained from Lonza (#PT-2501, Vervier, Belgium). The cells were grown in a CTS™ StemPro® MSC SFM medium (A10332-01, Life Technologies) supplemented with glutamine as requested by the manufacturer.

The cells were cultivated for 10 days and examined during this period: the cultures were examined under the microscope and cells were counted after detachment from the support. It was observed that the cells proliferated well during this period on the peptide-treated surface while they did not proliferate and finally degenerate on a TCT surface.

## Claims

1. A method for coating a solid support which is suitable for being used in cell culture applications, comprising:
(a) preparing a solution of at least one carrier protein comprising, either attached thereto or as a part of its amino acid sequence, at least one cell function-modulating peptide sequence, wherein said cell function-modulating peptide sequence comprises a cell-binding peptide motif, and wherein said carrier protein is an albumin or a protein derived from an albumin;
(b) heating the solution to at least 50°C and incubating same under conditions which result in the formation of soluble carrier protein aggregates comprising between 2 and 1000 protein molecules;
(c) contacting the carrier protein aggregates with the solid support under conditions that allow for the non-covalent adsorption of the aggregates to the support;
(d) optionally, washing the solid support to remove unbound carrier protein and carrier protein aggregates;
whereby a solid support is provided that comprises on its surface a plurality of cell function-modulating peptide sequences.

2. Method according to claim 1, wherein the surface of the solid support is hydrophobic or hydrophilic.

3. Method according to claims 1-2, wherein 2, 3, 4, 5 or more different cell function-modulating peptide sequences are used, either in the same or in different carrier protein.

4. Method according to claims 1-3, wherein the protein aggregates comprise between 5 and 100 protein molecules.

5. Method according to claims 1-4, wherein in step (b) the solution is heated to a temperature between 50°C and 75°C, preferably, between 65°C and 73°C, and more preferably between 68°C and 72°C.

6. Method according to claims 1-5, wherein in step (b) the conditions are selected so as to obtain at least 50%, preferably at least 90% of the protein aggregate in the soluble form.

7. Method according to claims 1-6, wherein the cell-binding peptide motif is an RGD motif and/or a cell-binding peptide motif selected from the sequences shown in SEQ ID NO:1-26.

8. Method according to claim 7, wherein the at least one cell function-modulating peptide sequence further comprises a peptide sequence with growth factor activity selected from SEQ ID NO:27-56

9. Method according to claim 1-8, wherein cell function-modulating peptides are comprised by the carrier proteins so as to form a molecular mosaic of cell function-modulating peptides which are present on or in the different carrier proteins of the aggregate.

10. Coated solid support obtainable by a method according to claims 1-9.

11. Coated solid support having non-covalently adsorbed on its surface at least one aggregate of carrier proteins, wherein said carrier proteins are albumins or proteins derived from albumins and comprise, either attached thereto or as a part of their amino acid sequence, at least one cell function-modulating peptide sequence, said cell function-modulating peptide sequence comprising a cell-binding peptide motif; and wherein said aggregate comprises between 2 and 1000 protein molecules.

12. Coated solid support according to claim 11, wherein the surface of said support comprises 2 or more different cell-function-modulating peptide sequences, each of which comprises a cell-binding peptide motif.

13. Coated solid support according to claims 11-12, wherein less than 30% of the carrier proteins are removed from the coated surface after incubation for 24h at 37°C.

14. Coated solid support according to claims 11-13, wherein the cell-binding peptide motif is an RGD motif and/or a cell-binding peptide motif selected from the sequences shown in SEQ ID NO:1-26.

15. Coated solid support according to claim 14, wherein the at least one cell function-modulating peptide sequence further comprises a peptide sequence with growth factor activity selected from SEQ ID NO:27-56.

16. Coated solid support according to claims 11-15, wherein the surface of said support comprises a molecular mosaic of cell-function modulating peptide sequences which are present on or in the different carrier proteins of the aggregate.

## Patentansprüche

1. Verfahren zum Beschichten eines festen Trägers, der zur Verwendung in Zellkulturanwendungen geeignet ist, wobei das Verfahren Schritte umfasst, bei denen man:
(a) eine Lösung aus mindestens einem Trägerprotein herstellt, das entweder daran gebunden oder als Teil seiner Aminosäuresequenz mindestens eine Zellfunktion-modulierende Peptidsequenz umfasst, wobei die Zellfunktion-modulierende Peptidsequenz ein Zell-bindendes Peptid-Motiv umfasst, und wobei das Trägerprotein ein Albumin ist oder ein Protein, das von einem Albumin abgeleitet ist;
(b) die Lösung auf mindestens 50°C erwärmt und diese unter Bedingungen inkubiert, die zur Bildung von löslichen Trägerproteinaggregaten führen, welche zwischen 2 und 1000 Proteinmoleküle umfassen;
(c) die Trägerproteinaggregate mit dem festen Träger unter Bedingungen in Kontakt bringt, welche die nichtkovalente Adsorption der Aggregate an den Träger ermöglichen;
(d) gegebenenfalls den festen Träger wäscht, um ungebundenes Trägerprotein und Trägerproteinaggregate zu entfernen;
wodurch ein fester Träger bereitgestellt wird, der auf seiner Oberfläche eine Vielzahl von Zellfunktion-modulierenden Peptid-Sequenzen umfasst.

2. Verfahren nach Anspruch 1, wobei die Oberfläche des festen Trägers hydrophob oder hydrophil ist.

3. Verfahren nach den Ansprüchen 1-2, wobei 2, 3, 4, 5 oder mehr verschiedene Zellfunktion-modulierende Peptid-Sequenzen entweder im gleichen oder in verschiedenen Trägerproteinen verwendet werden.

4. Verfahren nach den Ansprüchen 1-3, wobei die Proteinaggregate zwischen 5 und 100 Proteinmoleküle umfassen.

5. Verfahren nach den Ansprüchen 1-4, wobei in Schritt (b) die Lösung auf eine Temperatur zwischen 50°C und 75°C, vorzugsweise zwischen 65°C und 73°C, und besonders bevorzugt zwischen 68°C und 72°C erwärmt wird.

6. Verfahren nach den Ansprüchen 1-5, wobei in Schritt (b) die Bedingungen so ausgewählt werden, dass mindestens 50%, vorzugsweise mindestens 90%, der Proteinaggregate in löslicher Form erhalten werden.

7. Verfahren nach den Ansprüchen 1-6, wobei das Zell-bindende Peptid-Motiv ein RGD-Motiv und/oder ein Zell-bindendes Peptid-Motiv ausgewählt aus den in SEQ ID NO:1-26 gezeigten Sequenzen ist.

8. Verfahren nach Anspruch 7, wobei die mindestens eine Zellfunktion-modulierende Peptid-Sequenz ferner eine Peptid-Sequenz mit Wachstumsfaktor-Aktivität ausgewählt aus SEQ ID NO:27-56 umfasst.

9. Verfahren nach den Ansprüchen 1-8, wobei Zellfunktion-modulierende Peptide von den Trägerproteinen so umfasst sind, dass ein molekulares Mosaik aus Zellfunktion-modulierenden Peptiden gebildet wird, die auf oder in den verschiedenen Trägerproteinen des Aggregats vorliegen.

10. Beschichteter fester Träger, erhältlich nach einem Verfahren gemäß den Ansprüchen 1-9.

11. Beschichteter fester Träger, auf dessen Oberfläche mindestens ein Aggregat aus Trägerproteinen nicht-kovalent adsorbiert ist, wobei die Trägerproteine Albumine oder von Albuminen abgeleitete Proteine sind und entweder daran gebunden oder als Teil ihrer Aminosäuresequenz mindestens eine Zellfunktion-modulierende Peptid-Sequenz umfassen, wobei die Zellfunktion-modulierende Peptid-Sequenz ein Zell-bindendes Peptid-Motiv umfasst; und wobei das Aggregat zwischen 2 und 1000 Proteinmoleküle umfasst.

12. Beschichteter fester Träger nach Anspruch 11, wobei die Oberfläche des Trägers 2 oder mehrere verschiedene Zellfunktion-modulierende Peptid-Sequenzen umfasst, von denen jede ein Zell-bindendes Peptid-Motiv umfasst.

13. Beschichteter fester Träger nach den Ansprüchen 11-12, wobei weniger als 30% der Trägerproteine nach 24-stündiger Inkubation bei 37°C von der beschichteten Oberfläche entfernt werden.

14. Beschichteter fester Träger nach den Ansprüchen 11-13, wobei das Zell-bindende Peptid-Motiv ein RGD-Motiv und/oder ein Zell-bindendes Peptid-Motiv ausgewählt aus den in SEQ ID NO:1-26 gezeigten Sequenzen ist.

15. Beschichteter fester Träger nach Anspruch 14, wobei die mindestens eine Zellfunktion-modulierende Peptid-Sequenz ferner eine Peptid-Sequenz mit Wachstumsfaktor-Aktivität ausgewählt aus SEQ ID NO:27-56 umfasst.

16. Beschichteter fester Träger nach den Ansprüchen 11-15, wobei die Oberfläche des Trägers ein molekulares Mosaik aus Zellfunktion-modulierenden Peptiden umfasst, die auf oder in den verschiedenen Trägerproteinen des Aggregats vorliegen.

## Revendications

1. Procédé de revêtement d'un support solide approprié pour être utilisé dans des applications de culture de cellules, lequel procédé comporte les étapes suivantes :
a) préparer une solution d'au moins une protéine vectrice comportant, attachée à elle-même ou en tant que partie de sa séquence d'acides aminés, au moins une séquence peptidique modulant une fonction cellulaire, laquelle séquence peptidique modulant une fonction cellulaire comprend un motif peptidique de liaison à une cellule, et laquelle protéine vectrice est une albumine ou une protéine dérivée d'une albumine,
b) chauffer cette solution à au moins 50 °C et la faire incuber dans des conditions qui provoquent la formation d'agrégats solubles de protéine vectrice comprenant de 2 à 1000 molécules de protéine,
c) et mettre ces agrégats de protéine vectrice en contact avec le support solide dans des conditions qui permettent aux agrégats de s'adsorber sur le support par voie non-covalente,
d) et en option, laver le support solide pour en chasser les molécules et agrégats de protéine vectrice non lié(e)s,
ce par quoi l'on obtient un support solide qui porte à sa surface de multiples séquences peptidiques modulant une fonction cellulaire.

2. Procédé conforme à la revendication 1, dans lequel la surface du support solide est hydrophobe ou hydrophile.

3. Procédé conforme à la revendication 1 ou 2, dans lequel on utilise deux, trois, quatre ou cinq, voire plus, différentes séquences peptidiques modulant une fonction cellulaire, portées par la même protéine vectrice ou par des protéines vectrices différentes.

4. Procédé conforme à l'une des revendications 1 à 3, dans lequel les agrégats de protéine comportent de 5 à 100 molécules de protéine.

5. Procédé conforme à l'une des revendications 1 à 4, dans lequel, dans l'étape (b), on chauffe la solution à une température de 50 à 75 °C, de préférence de 65 à 73 °C, et mieux encore de 68 à 72 °C.

6. Procédé conforme à l'une des revendications 1 à 5, dans lequel, dans l'étape (b), on choisit des conditions appropriées pour obtenir au moins 50 %, et de préférence au moins 90 %, des agrégats de protéine sous la forme soluble.

7. Procédé conforme à l'une des revendications 1 à 6, dans lequel le motif peptidique de liaison à une cellule est un motif RGD et/ou un motif peptidique de liaison à une cellule choisi parmi les séquences présentées en tant que Séquences N° 1 à N° 26.

8. Procédé conforme à la revendication 7, dans lequel la séquence peptidique modulant une fonction cellulaire, au nombre d'au moins une, comporte en outre une séquence peptidique dotée d'une activité de facteur de croissance, choisie parmi les Séquences N° 27 à N° 56.

9. Procédé conforme à l'une des revendications 1 à 8, dans lequel les peptides modulant une fonction cellulaire sont portés par les protéines vectrices de telle manière que les peptides modulant une fonction cellulaire qui se trouvent sur ou dans les différentes protéines vectrices d'un agrégat forment une mosaïque moléculaire.

10. Support solide revêtu, accessible par un procédé conforme à l'une des revendications 1 à 9.

11. Support solide revêtu, portant à sa surface, adsorbé par voie non-covalente, au moins un agrégat de protéines vectrices, lesquelles protéines vectrices sont des albumines ou des protéines dérivées d'une albumine et comportent, attachées à elles-mêmes ou en tant que parties de leur séquence d'acides aminés, au moins une séquence peptidique modulant une fonction cellulaire, laquelle séquence peptidique modulant une fonction cellulaire comprend un motif peptidique de liaison à une cellule, et lequel agrégat comporte de 2 à 1000 molécules de protéine.

12. Support solide revêtu, conforme à la revendication 11, duquel support la surface porte deux différentes séquences peptidiques modulant une fonction cellulaire ou plus, dont chacune comprend un motif peptidique de liaison à une cellule.

13. Support solide revêtu, conforme à l'une des revendications 11 et 12, duquel moins de 30 % des protéines vectrices sont chassées de la surface revêtue après 24 heures d'incubation à 37 °C.

14. Support solide revêtu, conforme à l'une des revendications 11 à 13, dans lequel le motif peptidique de liaison à une cellule est un motif RGD et/ou un motif peptidique de liaison à une cellule choisi parmi les séquences présentées en tant que Séquences N° 1 à N° 26.

15. Support solide revêtu, conforme à la revendication 14, dans lequel la séquence peptidique modulant une fonction cellulaire, au nombre d'au moins une, comporte en outre une séquence peptidique dotée d'une activité de facteur de croissance, choisie parmi les Séquences N° 27 à N° 56.

16. Support solide revêtu, conforme à l'une des revendications 11 à 15, duquel support la surface porte une mosaïque moléculaire de séquences peptidiques modulant une fonction cellulaire qui se trouvent sur ou dans les différentes protéines vectrices de l'agrégat.


### SEQUENCE LISTING

<110> Eppendorf AG
<120> Method for coating a solid support
<130> P 98350
<160> 64
<170> PatentIn version 3.5
<210> 1
   <211> 5
   <212> PRT
   <213> artificial
<220>
   <223> cell function-modulating peptide, cyclic
<220>
   <221> MOD_RES
   <222> (1)..(1)
   <223> position 1 and 5 are connected to form a cyclic peptide
<220>
   <221> MOD_RES
   <222> (5)..(5)
   <223> position 1 and 5 are connected to form a cyclic peptide
<400> 1
   Arg Gly Asp Pro Cys
   1               5
<210> 2
   <211> 5
   <212> PRT
   <213> artificial
<220>
   <223> cell function-modulating peptide
<400> 2
   Gly Arg Gly Asp Ser
   1               5
<210> 3
   <211> 9
   <212> PRT
   <213> artificial
<220>
   <223> cell function-modulating peptide
<400> 3
   Gly Ser Gly Arg Gly Asp Ser Gly Ser
   1               5
<210> 4
   <211> 10
   <212> PRT
   <213> artificial
<220>
   <223> cell function-modulating peptide
<400> 4
   Val Thr Gly Arg Gly Asp Ser Pro Ala Ser
   1               5                   10
<210> 5
   <211> 15
   <212> PRT
   <213> artificial
<220>
   <223> cell function-modulating peptide
<400> 5
   Cys Gly Gly Asn Gly Glu Pro Arg Gly Asp Thr Tyr Arg Ala Tyr
   1               5                   10                  15
<210> 6
   <211> 12
   <212> PRT
   <213> artificial
<220>
   <223> cell function-modulating peptide, comprising a C-terminal NH2 group
<220>
   <221> MOD_RES
   <222> (12)..(12)
   <223> AMIDATION
<400> 6
   Gly Pro Arg Gly Asn Arg Gly Asp Ser Ile Asp Gln
   1               5                   10
<210> 7
   <211> 12
   <212> PRT
   <213> artificial
<220>
   <223> cell function-modulating peptide, comprising a C-terminal NH2 group
<220>
   <221> MOD_RES
   <222> (12)..(12)
   <223> AMIDATION
<400> 7
   Gly Ser Pro Gly Glu Arg Gly Asp Gln Gly Ala Arg
   1 5 10
<210> 8
   <211> 10
   <212> PRT
   <213> artificial
<220>
   <223> cell function-modulating peptide, comprising a C-terminal NH2 group
<220>
   <221> MOD_RES
   <222> (10)..(10)
   <223> AMIDATION
<400> 8
   Asp Gly Arg Gly Asp Ser Val Ala Tyr Gly
   1 5 10
<210> 9
   <211> 11
   <212> PRT
   <213> artificial
<220>
   <223> cell function-modulating peptide, comprising a C-terminal NH2 group
<220>
   <221> MOD_RES
   <222> (11)..(11)
   <223> AMIDATION
<400> 9
   Pro Arg Gly Asp Ser Gly Tyr Arg Gly Asp Ser
   1 5 10
<210> 10
   <211> 12
   <212> PRT
   <213> artificial
<220>
   <223> cell function-modulating peptide
<400> 10
   Cys Gly Gly Glu Pro Arg Gly Asp Thr Tyr Arg Ala
   1 5 10
<210> 11
   <211> 9
   <212> PRT
   <213> artificial
<220>
   <223> cell function-modulating peptide
<400> 11
   Cys Gly Pro Arg Gly Asp Thr Tyr Gly
   1 5
<210> 12
   <211> 15
   <212> PRT
   <213> artificial
<220>
   <223> cell function-modulating peptide, comprising N-terminal Acetylation
<220>
   <221> MOD_RES
   <222> (1)..(1)
   <223> ACETYLATION
<400> 12
   Lys Gly Gly Pro Gln Val Thr Arg Gly Asp Val Phe Thr Met Pro
   1 5 10 15
<210> 13
   <211> 19
   <212> PRT
   <213> artificial
<220>
   <223> cell function-modulating peptide
<400> 13
   Glu Glu Ile Gln Ile Gly His Ile Pro Arg Glu Asp Val Asp Tyr His
   1 5 10 15
   Leu Tyr Pro
<210> 14
   <211> 5
   <212> PRT
   <213> artificial
<220>
   <223> cell function-modulating peptide
<400> 14
   Glu Ile Leu Asp Val
   1 5
<210> 15
   <211> 20
   <212> PRT
   <213> artificial
<220>
   <223> cell function-modulating peptide
<400> 15
   Arg Tyr Val Val Leu Pro Arg Pro Val Cys Phe Glu Lys Gly Met Asn
   1               5                   10                  15
   Tyr Thr Val Arg
               20
<210> 16
   <211> 19
   <212> PRT
   <213> artificial
<220>
   <223> cell function-modulating peptide
<400> 16
   Cys Ser Arg Ala Arg Lys Gln Ala Ala Ser Ile Lys Val Ala Val Ser
   1               5                   10                  15
   Ala Asp Arg
<210> 17
   <211> 11
   <212> PRT
   <213> artificial
<220>
   <223> cell function-modulating peptide
<400> 17
   Gly Cys Gly Tyr Ile Gly Ser Arg Ser Pro Gly
   1               5                   10
<210> 18
   <211> 9
   <212> PRT
   <213> artificial
<220>
   <223> cell function-modulating peptide
<220>
   <221> MOD_RES
   <222> (3)..(3)
   <223> Xaa is hydroxyproline (Hyp)
<400> 18
   Gly Phe Xaa Gly Glu Arg Gly Val Gln
   1               5
<210> 19
   <211> 7
   <212> PRT
   <213> artificial
<220>
   <223> cell function-modulating peptide
<400> 19
   Ala Asp Gly Glu Ala Asp Pro
   1               5
<210> 20
   <211> 5
   <212> PRT
   <213> artificial
<220>
   <223> cell function-modulating peptide
<400> 20
   Pro His Ser Arg Asn
   1               5
<210> 21
   <211> 5
   <212> PRT
   <213> artificial
<220>
   <223> cell function-modulating peptide
<400> 21
   Leu His Ser Arg Asn
   1               5
<210> 22
   <211> 21
   <212> PRT
   <213> artificial
<220>
   <223> cell function-modulating peptide
<400> 22
   Arg Gly Asp Gly Gly Gly Gly Gly Gly Gly Gly Gly Gly Gly Gly Gly
   1 5 10 15
   Pro His Ser Arg Asn
   20
<210> 23
   <211> 21
   <212> PRT
   <213> artificial
<220>
   <223> cell function-modulating peptide
<400> 23
   Arg Gly Asp Gly Gly Gly Gly Gly Gly Gly Gly Gly Gly Gly Gly Gly
   1 5 10 15
   Leu His Ser Arg Asn
   20
<210> 24
   <211> 15
   <212> PRT
   <213> artificial
<220>
   <223> cell function-modulating peptide
<400> 24
   Gly Thr Pro Gly Pro Gln Gly Ile Ala Gly Gln Arg Gly Val Val
   1 5 10 15
<210> 25
   <211> 7
   <212> PRT
   <213> artificial
<220>
   <223> cell function-modulating peptide
<400> 25
   Met Asn Tyr Tyr Ser Asn Ser
   1 5
<210> 26
   <211> 10
   <212> PRT
   <213> artificial
<220>
   <223> cell function-modulating peptide, comprising a C-terminal NH2 group and N-terminal Acetylation
<220>
   <221> MOD_RES
   <222> (1)..(1)
   <223> ACETYLATION
<220>
   <221> MOD_RES
   <222> (10)..(10)
   <223> AMIDATION
<400> 26
   Cys Gly Gly Phe His Arg Arg Ile Lys Ala
   1               5                   10
<210> 27
   <211> 16
   <212> PRT
   <213> artificial
<220>
   <223> cell function-modulating peptide
<400> 27
   Thr Gly Gln Tyr Leu Ala Met Asp Thr Asp Gly Leu Leu Tyr Gly Ser
   1               5                   10                  15
<210> 28
   <211> 16
   <212> PRT
   <213> artificial
<220>
   <223> cell function-modulating peptide
<400> 28
   Ala Asn Arg Tyr Leu Ala Met Lys Glu Asp Gly Arg Leu Leu Ala Ser
   1               5                   10                  15
<210> 29
   <211> 15
   <212> PRT
   <213> artificial
<220>
   <223> cell function-modulating peptide
<400> 29
   Glu Val Tyr Val Val Ala Glu Asn Gln Gln Gly Lys Ser Lys Ala
   1               5                   10                  15
<210> 30
   <211> 9
   <212> PRT
   <213> artificial
<220>
   <223> cell function-modulating peptide
<400> 30
   Val Val Asn Gly Ile Pro Thr Arg Asn
   1 5
<210> 31
   <211> 7
   <212> PRT
   <213> artificial
<220>
   <223> cell function-modulating peptide
<400> 31
   Lys Gln Leu Arg Ala Met Cys
   1 5
<210> 32
   <211> 11
   <212> PRT
   <213> artificial
<220>
   <223> cell function-modulating peptide
<400> 32
   Pro Ala Ser Asp Ala Phe Gln Arg Lys Leu Glu
   1 5 10
<210> 33
   <211> 6
   <212> PRT
   <213> artificial
<220>
   <223> cell function-modulating peptide
<400> 33
   Leu Ala Arg Leu Leu Thr
   1 5
<210> 34
   <211> 14
   <212> PRT
   <213> artificial
<220>
   <223> cell function-modulating peptide
<400> 34
   Ser Phe Leu Leu Arg Asn Pro Asn Asp Lys Tyr Glu Pro Phe
   1 5 10
<210> 35
   <211> 37
   <212> PRT
   <213> artificial
<220>
   <223> cell function-modulating peptide
<400> 35
   Leu Leu Gly Asp Phe Phe Arg Lys Ser Lys Glu Lys Ile Gly Lys Glu
   1 5 10 15
   Phe Lys Arg Ile Val Gln Arg Ile Lys Asp Phe Leu Arg Asn Leu Val
   20 25 30
   Pro Arg Thr Glu Ser
   35
<210> 36
   <211> 12
   <212> PRT
   <213> artificial
<220>
   <223> cell function-modulating peptide
<400> 36
   Cys Gly Thr Gly Tyr Gly Ser Ser Ser Arg Arg Cys
   1 5 10
<210> 37
   <211> 36
   <212> PRT
   <213> artificial
<220>
   <223> cell function-modulating peptide
<400> 37
   Ser Leu Glu Glu Glu Trp Ala Gln Val Glu Cys Glu Val Tyr Gly Arg
   1 5 10 15
   Gly Cys Pro Ser Gly Ser Leu Asp Glu Ser Phe Tyr Asp Trp Phe Glu
   20 25 30
   Arg Gln Leu Gly
   35
<210> 38
   <211> 18
   <212> PRT
   <213> artificial
<220>
   <223> cell function-modulating peptide
<400> 38
   Gly Ser Leu Asp Glu Ser Phe Tyr Asp Trp Phe Glu Arg Gln Leu Gly
   1               5                   10                  15
   Lys Lys
<210> 39
   <211> 10
   <212> PRT
   <213> artificial
<220>
   <223> cell function-modulating peptide
<400> 39
   Arg Lys Ile Glu Ile Val Arg Lys Lys Cys
   1               5                   10
<210> 40
   <211> 10
   <212> PRT
   <213> artificial
<220>
   <223> cell function-modulating peptide
<400> 40
   Ile Val Arg Lys Lys Cys Arg Lys Ile Glu
   1               5                   10
<210> 41
   <211> 12
   <212> PRT
   <213> artificial
<220>
   <223> cell function-modulating peptide, comprising a C-terminal NH2 group
<220>
   <221> MOD_RES
   <222> (12)..(12)
   <223> AMIDATION
<400> 41
   Ala Leu Lys Arg Gln Gly Arg Thr Leu Tyr Gly Phe
   1 5 10
<210> 42
   <211> 15
   <212> PRT
   <213> artificial
<220>
   <223> cell function-modulating peptide
<400> 42
   Gly Gln Gly Phe Ser Tyr Pro Tyr Lys Ala Val Phe Ser Thr Gln
   1 5 10 15
<210> 43
   <211> 8
   <212> PRT
   <213> artificial
<220>
   <223> cell function-modulating peptide
<400> 43
   Ala Pro Ser Gly His Tyr Lys Gly
   1 5
<210> 44
   <211> 7
   <212> PRT
   <213> artificial
<220>
   <223> cell function-modulating peptide
<400> 44
   Met Gln Leu Pro Leu Ala Thr
   1 5
<210> 45
   <211> 8
   <212> PRT
   <213> artificial
<220>
   <223> cell function-modulating peptide
<400> 45
   Ala Pro Ser Gly His Tyr Lys Gly
   1 5
<210> 46
   <211> 5
   <212> PRT
   <213> artificial
<220>
   <223> cell function-modulating peptide
<400> 46
   Cys Val Arg Ala Cys
   1 5
<210> 47
   <211> 24
   <212> PRT
   <213> artificial
<220>
   <223> cell function-modulating peptide
<400> 47
   Asn Phe Cys Leu Gly Pro Cys Pro Tyr Ile Trp Ser Leu Asp Thr Gln
   1 5 10 15
   Tyr Ser Lys Val Leu Ala Leu Tyr
   20
<210> 48
   <211> 19
   <212> PRT
   <213> artificial
<220>
   <223> cell function-modulating peptide
<400> 48
   Glu Cys Gly Leu Leu Pro Val Gly Arg Pro Asp Arg Asn Val Trp Arg
   1 5 10 15
   Trp Leu Cys
<210> 49
   <211> 10
   <212> PRT
   <213> artificial
<220>
   <223> cell function-modulating peptide
<400> 49
   Lys Pro Ser Ser Ala Pro Thr Gln Leu Asn
   1               5                   10
<210> 50
   <211> 11
   <212> PRT
   <213> artificial
<220>
   <223> cell function-modulating peptide
<400> 50
   Lys Ala Ile Ser Val Leu Tyr Phe Asp Asp Ser
   1               5                   10
<210> 51
   <211> 10
   <212> PRT
   <213> artificial
<220>
   <223> cell function-modulating peptide
<400> 51
   Ser Asn Val Ile Leu Lys Lys Tyr Arg Asn
   1               5                   10
<210> 52
   <211> 20
   <212> PRT
   <213> artificial
<220>
   <223> cell function-modulating peptide
<400> 52
   Gly Trp Gln Asp Trp Ile Ile Ala Pro Glu Gly Tyr Ala Ala Tyr Tyr
   1               5                   10                  15
   Cys Glu Gly Glu
               20
<210> 53
   <211> 20
   <212> PRT
   <213> artificial
<220>
   <223> cell function-modulating peptide
<400> 53
   Ala Ile Ser Val Leu Tyr Phe Asp Asp Ser Ser Asn Val Ile Leu Lys
   1               5                   10                  15
   Lys Tyr Arg Asn
               20
<210> 54
   <211> 10
   <212> PRT
   <213> artificial
<220>
   <223> cell function-modulating peptide
<400> 54
   Ile Val Arg Lys Lys Cys Arg Lys Ile Glu
   1               5                   10
<210> 55
   <211> 16
   <212> PRT
   <213> artificial
<220>
   <223> cell function-modulating peptide, cyclic, with NAc at position 1 and NH2 group at position 16
<220>
   <221> MOD_RES
   <222> (1)..(1)
   <223> ACETYLATION
<220>
   <221> MOD_RES
   <222> (16)..(16)
   <223> AMIDATION
<400> 55
   Cys Ser Arg Arg Gly Glu Leu Ala Ala Ser Arg Arg Gly Glu Leu Cys
   1               5                   10                  15
<210> 56
   <211> 9
   <212> PRT
   <213> artificial
<220>
   <223> cell function-modulating peptide, with C-terminal NH2 group
<220>
   <221> MOD_RES
   <222> (9)..(9)
   <223> AMIDATION
<400> 56
   Phe Pro Gln Ser Phe Leu Pro Arg Gly
   1 5
<210> 57
   <211> 609
   <212> PRT
   <213> Homo sapiens
<400> 57
<210> 58
   <211> 585
   <212> PRT
   <213> Artificial Sequence
<220>
   <223> HSA with loop 1 modified
<400> 58
   Asp Ala His Lys Ser Glu Val Ala His Arg Phe Lys Asp Leu Gly Glu
   1 5 10 15
   Glu Asn Phe Lys Ala Leu Val Leu Ile Ala Phe Ala Gln Tyr Leu Gln
   20 25 30
   Gln Cys Pro Phe Glu Asp His Val Lys Leu Val Asn Glu Val Thr Glu
   35 40 45
   Phe Ala Lys Thr Cys Val Ala Gly Arg Gly Asp Ser Asn Cys Asp Lys
   50 55 60
   Ser Leu His Thr Leu Phe Gly Asp Lys Leu Cys Thr Val Ala Thr Leu
   65 70 75 80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585
<210> 59
   <211> 585
   <212> PRT
   <213> Artificial Sequence
<220>
   <223> HSA with loop 2 modified
<400> 59
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Gly Arg Gly Asp Ser
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585
<210> 60
   <211> 585
   <212> PRT
   <213> Artificial Sequence
<220>
   <223> HSA with loop 3 modified
<400> 60
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gly Arg Gly Asp Ser Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
   580 585
<210> 61
   <211> 585
   <212> PRT
   <213> Artificial Sequence
<220>
   <223> HSA with loops 1-3 modified
<400> 61
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Gly Arg Gly Asp Ser Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Gly Arg Gly Asp Ser
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gly Arg Gly Asp Ser Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
   450 455 460
   Glu Lys Thr Pro Val Ser Asp Arg Val Thr Lys Cys Cys Thr Glu Ser
   465 470 475 480
   Leu Val Asn Arg Arg Pro Cys Phe Ser Ala Leu Glu Val Asp Glu Thr
   485 490 495
   Tyr Val Pro Lys Glu Phe Asn Ala Glu Thr Phe Thr Phe His Ala Asp
   500 505 510
   Ile Cys Thr Leu Ser Glu Lys Glu Arg Gln Ile Lys Lys Gln Thr Ala
   515 520 525
   Leu Val Glu Leu Val Lys His Lys Pro Lys Ala Thr Lys Glu Gln Leu
   530 535 540
   Lys Ala Val Met Asp Asp Phe Ala Ala Phe Val Glu Lys Cys Cys Lys
   545 550 555 560
   Ala Asp Asp Lys Glu Thr Cys Phe Ala Glu Glu Gly Lys Lys Leu Val
   565 570 575
   Ala Ala Ser Gln Ala Ala Leu Gly Leu
   580 585
<210> 62
   <211> 585
   <212> PRT
   <213> Artificial Sequence
<220>
   <223> HSA with linear stretch modified
<400> 62
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Arg Glu Asp Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
   325 330 335
   Arg His Pro Asp Tyr Ser Val Val Leu Leu Leu Arg Leu Ala Lys Thr
   340 345 350
   Tyr Glu Thr Thr Leu Glu Lys Cys Cys Ala Ala Ala Asp Pro His Glu
   355 360 365
   Cys Tyr Ala Lys Val Phe Asp Glu Phe Lys Pro Leu Val Glu Glu Pro
   370 375 380
   Gln Asn Leu Ile Lys Gln Asn Cys Glu Leu Phe Glu Gln Leu Gly Glu
   385 390 395 400
   Tyr Lys Phe Gln Asn Ala Leu Leu Val Arg Tyr Thr Lys Lys Val Pro
   405 410 415
   Gln Val Ser Thr Pro Thr Leu Val Glu Val Ser Arg Asn Leu Gly Lys
   420 425 430
   Val Gly Ser Lys Cys Cys Lys His Pro Glu Ala Lys Arg Met Pro Cys
   435 440 445
   Ala Glu Asp Tyr Leu Ser Val Val Leu Asn Gln Leu Cys Val Leu His
   450 455 460
   Glu Lys Thr Pro Val Ser Asp Arg Val Thr Lys Cys Cys Thr Glu Ser
   465 470 475 480
   Leu Val Asn Arg Arg Pro Cys Phe Ser Ala Leu Glu Val Asp Glu Thr
   485 490 495
   Tyr Val Pro Lys Glu Phe Asn Ala Glu Thr Phe Thr Phe His Ala Asp
   500 505 510
   Ile Cys Thr Leu Ser Glu Lys Glu Arg Gln Ile Lys Lys Gln Thr Ala
   515 520 525
   Leu Val Glu Leu Val Lys His Lys Pro Lys Ala Thr Lys Glu Gln Leu
   530 535 540
   Lys Ala Val Met Asp Asp Phe Ala Ala Phe Val Glu Lys Cys Cys Lys
   545 550 555 560
   Ala Asp Asp Lys Glu Thr Cys Phe Ala Glu Glu Gly Lys Lys Leu Val
   565 570 575
   Ala Ala Ser Gln Ala Ala Leu Gly Leu
               580                 585
<210> 63
   <211> 585
   <212> PRT
   <213> Artificial Sequence
<220>
   <223> HSA with inversed loop modified
<400> 63
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln Arg Tyr Val Val Leu Pro Arg Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
   195 200 205
   Arg Ala Phe Lys Ala Trp Ala Val Ala Arg Leu Ser Gln Arg Phe Pro
   210 215 220
   Lys Ala Glu Phe Ala Glu Val Ser Lys Leu Val Thr Asp Leu Thr Lys
   225 230 235 240
   Val His Thr Glu Cys Cys His Gly Asp Leu Leu Glu Cys Ala Asp Asp
   245 250 255
   Arg Ala Asp Leu Ala Lys Tyr Ile Cys Glu Asn Gln Asp Ser Ile Ser
   260 265 270
   Ser Lys Leu Lys Glu Cys Cys Glu Lys Pro Leu Leu Glu Lys Ser His
   275 280 285
   Cys Ile Ala Glu Val Glu Asn Asp Glu Met Pro Ala Asp Leu Pro Ser
   290 295 300
   Leu Ala Ala Asp Phe Val Glu Ser Lys Asp Val Cys Lys Asn Tyr Ala
   305 310 315 320
   Glu Ala Lys Asp Val Phe Leu Gly Met Phe Leu Tyr Glu Tyr Ala Arg
   325 330 335
   Arg His Pro Asp Tyr Ser Val Val Leu Leu Leu Arg Leu Ala Lys Thr
   340 345 350
   Tyr Glu Thr Thr Leu Glu Lys Cys Cys Ala Ala Ala Asp Pro His Glu
   355 360 365
   Cys Tyr Ala Lys Val Phe Asp Glu Phe Lys Pro Leu Val Glu Glu Pro
   370 375 380
   Gln Asn Leu Ile Lys Gln Asn Cys Glu Leu Phe Glu Gln Leu Gly Glu
   385 390 395 400
   Tyr Lys Phe Gln Asn Ala Leu Leu Val Arg Tyr Thr Lys Lys Val Pro
   405 410 415
   Gln Val Ser Thr Pro Thr Leu Val Glu Val Ser Arg Asn Leu Gly Lys
   420 425 430
   Val Gly Ser Lys Cys Cys Lys His Pro Glu Ala Lys Arg Met Pro Cys
   435 440 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585
<210> 64
   <211> 585
   <212> PRT
   <213> Artificial Sequence
<220>
   <223> HSA with inversed loop, linear stretch and loop 3 modified
<400> 64
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln Arg Tyr Val Val Leu Pro Arg Leu
               100                 105                 110
   Arg Glu Asp Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gly Arg Gly Asp Ser Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585